# EUROPÄISCHE PATENTSCHRIFT

(11) **EP 1 086 075 B1**
(45) Veröffentlichungstag und Bekanntmachung des Hinweises auf die Patenterteilung: **29.10.2003**
(21) Anmeldenummer: 99926503.6
(22) Anmeldetag: 02.06.1999
(51) Int. Cl.: C07C 263/20

(54) **VERFAHREN ZUR AUFARBEITUNG VON DESTILLATIONSRÜCKSTÄNDEN AUS DER SYNTHESE VON TOLUYLENDIISOCYANAT**
METHOD FOR TREATING DISTILLATION RESIDUES FROM THE SYNTHESIS OF TOLUENE DIISOCYANATE
PROCEDE DE TRAITEMENT DE RESIDUS DE DISTILLATION RESULTANT DE LA SYNTHESE DE DIISOCYANATE DE TOLUYLENE

(30) Priorität: 18.06.1998 DE 19827086
(43) Veröffentlichungstag der Anmeldung: 28.03.2001
(73) Patentinhaber: BASF AKTIENGESELLSCHAFT, 67056 Ludwigshafen (DE)
(72) Erfinder: SCHWARZ, Hans, Volkmar, B-1410 Waterloo (BE); MAURER, Markus, D-67071 Ludwigshafen (DE); SANDER, Michael, D-01945 Ruhland (DE); STRÖFER, Eckhard, D-68163 Mannheim (DE); LEUTHOLD, Rene, D-01945 Hohenbocka (DE); HANTEL, Burghard, D-01936 Cosel (DE); RICHTER, Siegfried, D-04928 Schraden (DE); THERRE, Jörg, D-67550 Worms (DE)
(86) Internationale Anmeldenummer: EP9903812
(87) Internationale Veröffentlichungsnummer: WO99065868

(56) Entgegenhaltungen:
- EP-A- 0 017 972
- US-A- 3 499 035
- US-A- 4 654 443

## Beschreibung

Die Erfindung betrifft ein Verfahren zur Aufarbeitung von Destillationsrückständen aus der Synthese von Toluylendiisocyanat.

Toluylendiisocyanat (TDI) wird in großen Mengen zur Herstellung von Polyurethanen eingesetzt. Die Herstellung von TDI erfolgt zumeist durch Umsetzung von Toluylendiamin (TDA) mit Phosgen. Dieses Verfahren ist seit langem bekannt und vielfach in der Literatur beschrieben.

Üblicherweise wird dazu das TDA mit Phosgen in einer konventionellen zweistufigen Phosgenierung umgesetzt.

Es gibt jedoch auch andere Synthesen, in denen TDI durch Spaltung eines Urethans hergestellt wird, wobei das Urethan aus TDA, Harnstoff und Alkohol oder auf einem anderen Wege synthetisiert wird.

In allen diesen Fällen steht am Ende der Synthese ein Destillationsschritt, in dem das TDI von Nebenprodukten abgetrennt wird. Das Verhältnis von TDI zu Rückstand aus diesem Destillationsschritt kann zwischen 1 und 20 % liegen. Es besteht also ein erheblicher wirtschaftlicher Anreiz, diesen Rückstand stofflich zu verwerten.

Im Stand der Technik sind verschiedene Verfahren zur direkten stofflichen Verwertung von Rückständen aus der TDI-Herstellung beschrieben.

In US-A-3,499,021 wird der Rückstand phosgeniert und in das Verfahren zurückgeführt. In den Patenten DE-A-42 11 774, DD-A-257 827 und US-A-3,694,323 wird der Rückstand mit MDI versetzt, teilweise destilliert und zu einem Polyurethan umgesetzt. Die direkte Umsetzung des Rückstandes mit einem Polyol zum entsprechenden Polyurethan wird in den Patenten DD-A-296 088, US A-4,143,008 und US-A-4,000,099 beschrieben. Diese Verfahren führen jedoch zu minderwertigen Produkten, die bei der Herstellung von Polyurethanen zumeist nicht einsetzbar sind.

Eine weitere Möglichkeit der Verwertung des Rückstandes ist dessen Umsetzung mit Wasser, die sogenannte Hydrolyse. Derartige Verfahren sind ebenfalls vielfach beschrieben. Die Hydrolyse des Rückstandes wird durch Basen bzw. Säuren begünstigt. Auch Amine begünstigen die Hydrolyse. Die Hydrolyse kann dazu genutzt werden, den TDI-Destillationsrückstand zu Denaturieren, wie beispielsweise in US-A-4,091,009 beschrieben. Eine weitere Möglichkeit ist die Rückgewinnung von TDA, das dann wieder mit Phosgen zu TDI umgesetzt werden kann. Derartige Verfahren werden beispielsweise in DE-A-29 42 678, JP-A-5 8201 751 und DE-A-19 62 598 beschrieben.

Allen aufgeführten Patenten ist gemeinsam, daß sie Batchprozesse beschreiben, bei denen zunächst sehr schnell aus dem TDI-Rückstand und Wasser eine feste Phase entsteht, die sich bei der weiteren Umsetzung langsam wieder verflüssigt. Diese Feststoffbildung kann zu erheblichen Störungen bei der Reaktionsführung führen.

In DE-A-27 03 313 wird eine Hydrolyseverfahren beschrieben, das sowohl diskontinuierlich in einem Autoklaven als auch kontinuierlich in einem Röhrenreaktor durchgeführt wird. Die Hydrolyse des festen TDI-Rückstandes wird mit wäßriger Ammoniaklösung, Lösungen von primärem oder sekundären Aminen in Wasser oder wäßriger TDA-Lösungen durchgeführt. Die Verwendung von wäßrigen TDA-Lösungen wird dabei als weniger bevorzugt bezeichnet. Das Verfahren gemäß DE-A-27 03 313 hat jedoch auch Nachteile. So führt die Verwendung von Ammoniaklösung zur Bildung von Salzen, beispielsweise Ammoniumbicarbonat, Ammoniumcarbonat und Salze von organischen Polyaminen, die thermisch gespalten oder auf andere Weise abgetrennt werden müssen. Die zugesetzten primären oder sekundären Amine müssen vom rückgewonnenen TDA abgetrennt werden. Bei der Verwendung von wäßriger TDA-Lösung müssen Löslichkeitsvermittler zugesetzt werden, die nach der Hydrolyse vom Hydrolysat abgetrennt werden müssen.

In US-A-3,499,035 wird ein Hydrolyseverfahren beschrieben, bei dem der TDI-Rückstand zunächst mit Wasser teilweise hydrolysiert und das so erhaltene feste Zwischenprodukt in einer zweiten Verfahrensstufe mit TDA umgesetzt wird. Bei diesem Verfahren kommt es in der ersten Verfahrensstufe zu einer starken Feststoffbildung.

US-A-4,654,443 beschreibt ein Hydrolyseverfahren, bei dem in einem ersten Verfahrensschritt der TDI-Rückstand mit TDA zu einem Feststoff umgesetzt und in einem zweiten Schritt dieses Zwischenprodukt mit Wasser hydrolysiert wird. Nachteilig ist auch hier, daß das Verfahren zwei Verfahrensschritte umfaßt, und daß TDA dem Reaktionsgemisch zugesetzt werden muß. Außerdem kommt es auch hier zu einer starken Ausbildung von Feststoffen.

In JP-A-151 270/97 wird ein Verfahren zur Hydrolyse von TDI-Rückständen mit überkritischem oder sehr heißem Wasser beschrieben. Nachteilig bei diesem Verfahren ist der sehr hohe Druck, der die Verwendung spezieller Apparate notwendig macht, sowie die Korrosionsprobleme, die sich aus der Verwendung von überkritischem Wasser ergeben. Außerdem muß mit einem hohen Wasserüberschuß gearbeitet werden.

Aufgrund der beschriebenen Probleme wurde die Hydrolyse von TDI-Destillationsrückstand bisher noch nicht großtechnisch realisiert. Derzeit ist es noch immer so, daß der Großteil der Destillationsrückstände verbrannt werden muß, was sich sehr nachteilig auf die Wirtschaftlichkeit der TDI-Hersteilung auswirkt.

Aufgabe der vorliegenden Erfindung war es daher, ein Verfahren zur stofflichen Verwertung von TDI-Rückständen mittels Hydrolyse zu finden, das betriebssicher ist, in hoher Ausbeute zu verwertbaren Produkten, insbesondere TDA, führt, und ohne Probleme mit vorhandenen Anlagen zur Herstellung von TDI kombiniert werden kann.

Überraschenderweise wurde gefunden, daß es bei der Hydrolyse von TDI-Rückständen zu keiner Bildung von Feststoffen kommt, wenn die Hydrolyse des eingesetzten TDI-Destillationsrückstandes in einem kontinuierlichen oder halbkontinuierlichen Verfahren im rückvermischten System bei gleichzeitiger Gegenwart von Hydrolysat und von Wasser durchgeführt wird.

Gegenstand der vorliegenden Erfindung ist demzufolge ein Verfahren zur Hydrolyse von TDI-Destillationsrückständen, das dadurch gekennzeichnet ist, daß man den eingesetzten TDI-Destillationsrückstand in einem kontinuierlichen oder halbkontinuierlichen Verfahren in einem rückvermischten Reaktor in Gegenwart von Hydrolysat mit Wasser umsetzt. Dabei kommt es zu einer Umsetzung des Destillationsrückstands zu TDA und Kohlendioxid. Hierbei wird nicht nur das freie TDI des TDI-Destillatiönsrückstandes zu TDA umgesetzt, sondern es kommt überraschenderweise auch zu einer weitgehenden Spaltung der anderen Bestandteile des TDI-Destillationsrückstandes, was zu einer sehr hohen Ausbeute an TDA führt.

Als Hydrolysat werden hierbei die Umsetzungsprodukte des TDI-Rückstandes mit Wasser verstanden.

Die Hydrolyse wird bei gleichzeitiger Anwesenheit von Hydrolysat und Wasser durchgeführt. Bei einer solchen Verfahrensweise kann überraschenderweise die Bildung von Feststoffen völlig vermieden werden. Dies kann vorzugsweise durch eine intensive Rückvermischung des Reaktionsgemisches bewirkt werden.

Die Hydrolyse wird vorzugsweise bei Temperaturen im Bereich von 120 bis 250°C und Drücken im Bereich von 1 bis 50 bar durchgeführt. Der Druck sollte vorzugsweise etwas höher gewählt werden als der Siededruck des Austrags aus dem Hydrolysereaktor bei Reaktionstemperatur. Das Masseverhältnis von TDI-Rückstand zu Wasser beträgt vorzugsweise 4,8:1 bis 1:5, vorzugsweise 1:1,0 bis 1:3.

Um eine Feststoffbildung beim Reaktionsstart auszuschließen, wird im Reaktionsbehälter Hydrolysat vorgelegt, das gegebenenfalls in einem separaten Reaktor vorher produziert werden kann.

Besonders geeignete Ausführungsformen des erfindungsgemäßen Verfahrens sind das Semibatch-Verfahren und insbesondere das kontinuierliche Verfahren. Hierbei ist, wie oben ausgeführt, eine gute Rückvermischung des Reaktionsgemisches zu gewährleisten, um eine Bildung von Feststoffen zuverlässig auszuschließen.

Beim Semibatch-Verfahren erfolgt die Vorlage von Hydrolysat und gleichzeitige Zudosierung von Wasser und TDI-Destillationsrückstand in einen rückvermischten Reaktor, z.B. einen Rührkessel.

Beim kontinuierlichen Verfahren erfolgt die gleichzeitige Zudosierung von TDI-Destillationsrückstand und Wasser in einen kontinuierlich durchströmten, rückvermischten Reaktor, z.B. einen Rührkessel, eine Strahlschlaufe, eine Reaktionsmischpumpe, einen Umpumpkreis mit statischem Mischer und/oder Zweistoffmischdüse.

Zur Verbesserung der Reaktionsausbeute kann der kontinuierlich durchströmte, rückvermischte Reaktor auch als Reaktorkaskade bzw. als Kombination aus rückvermischtem Vorreaktor und nicht rückvermischtem Nachreaktor, z.B. als Rührkessel mit nachgeschaltetem Rohrreaktor, ausgebildet sein.

In einer besonders vorteilhaften Ausführungsform der vorliegenden Erfindung erfolgt die Zudosierung des TDI-Destillationsrückstandes nicht in die Gasphase des Reaktors, sondern getaucht in die Flüssigphase. Um Verstopfungen an der Einleitstelle zu vermeiden, muß im Einleitungsrohr eine ausreichende Strömungsgeschwindigkeit, vorzugsweise größer 0,5 m/s, herrschen.

Der TDI-Destillationsrückstand besteht zumeist aus 0 bis 80 Gew.-% TDI, Harnstoffen, teerartigen Oligomeren des TDI, Urethanen, Isocyanuraten, Biureten, Allophanaten, Uretdionen, Carbodiimiden, Urethaniminen und anderen Nebenprodukten.

Der Rückstand, der für die Hydrolyse eingesetzt wird, kann dem Sumpf der letzten Aufarbeitungskolonne oder nachgeschalteten Eindampfvorrichtungen entnommen werden. Bei den Eindampfvorrichtungen kann es sich beispielsweise um Fallfilmverdampfer gemäß NL-A-7109730, eine Dünnschichtverdampfer gemäß FR-A-2320-938, ein beheiztes Rohr mit Flashtopf gemäß DT-A-27 25 886, Rührkessel gemäß US-A-3,405,040, eine beheizte Kugelmühle gemäß US-A-3,457,291, einen ein- oder zweiwelligen Extruder gemäß EP-A-38 502, EP-A-463, eine Wirbelschicht gemäß DT-A-29 15 830, Schaufeltrockner, ein Zweiphasenwendelrohr oder einen beliebigen anderen Apparat handeln. Weder der TDI-Gehalt noch die Konsistenz des TDI-Rückstandes, d.h. ob er flüssig oder fest ist, und somit auch die Art der Eindampfvorrichtung schränken die Anwendbarkeit des erfindungsgemäßen Hydrolyseverfahrens ein. Zur besseren Handhabbarkeit des TDI-Rückstands ist es jedoch vorteilhaft, wenn er pumpfähig ist.

Zur weiteren Verbesserung der Handhabbarkeit des TDI-Destillationsrückstandes kann dieser auch in einem geeigneten, organischen Lösemittel, das mit dem Rückstand nicht reagiert, wie z.B. Toluol, N-Methylpyrolidon, DMF, Monochlorbenzol, Dichlorbenzol und anderen, aufgenommen werden.

Die Hydrolyse kann durch die Mitverwendung von Basen, wie Alkaliund/oder Erdalkalihydroxiden, z.B. Natronlauge, Kalilauge, Magnesiumhydroxid, Calciumhydroxid, Magnesiumoxid, Calciumoxid, Ammoniak oder Amine, sowie Säuren, wie Salzsäure, Bromwasserstoffsäure, oder Schwefelsäure begünstigt werden.

Bei Verwendung von Alkalihydroxiden werden diese vorzugsweise in einer Menge von 0,5 bis 5,0 Gew.-%, bezogen auf das Reaktionsgemisch, eingesetzt.

Die Verwendung von Katalysatoren, wie sie üblicherweise zur Spaltung von Urethanen eingesetzt werden, z.B. Salze von Eisen, Zink, Zinn und Zirkon, begünstigen ebenfalls die Hydrolyse.

Das Hydrolysat wird dem Hydrolysereaktor kontinuierlich entnommen und aufgearbeitet. Beim bevorzugten lösungsmittelfreien Verfahren ist das Hydrolysat einphasig. Zunächst erfolgt die destillative Abtrennung des im Überschuß eingesetzten Wassers und anschließend die destillative Abtrennung des TDA. Bei der Verwendung von wasserlöslichen Lösungsmitteln kann ein Großteil des Wassers bereits durch eine vorgeschaltete Phasentrennung abgetrennt werden. Das abgetrennte Wasser kann in jedem Fall in die Hydrolyse zurückgeführt werden.

Das durch die Hydrolyse gewonnene TDA kann nach entsprechender Reinigung und Aufarbeitung entweder dem Phosgenierungsreaktor des TDI-Prozesses zugesetzt oder dem Reaktionsgemisch, das bei der TDA-Herstellung durch Hydrierung von Dinitrotouol den Hydrierreaktor verläßt, vor der Aufarbeitung zum reinen TDA zugeführt werden. Die letztgenannte Verfahrensvariante hat den Vorteil, daß der Aufarbeitungsschritt nach der Hydrolyse vereinfacht oder gegebenenfalls ganz eingespart werden kann. Die Nebenprodukte der Hydrolyse können dann im Verfahrensschritt der TDA-Aufarbeitung mit dem TDA-Teer aus dem Verfahren ausgeschleust werden.

Alternativ kann der Hydrolyseaustrag nach der Abtrennung von Wasser ohne weitere Aufarbeitung oder nach der Abtrennung von Nebenprodukten auch zur Herstellung von Polyetherolen durch Anlagerung von Alkylenoxiden verwendet werden. Es ist natürlich auch möglich, das gereinigte TDA mit Alkylenoxiden zu Polyetherolen umzusetzen.

Die Erfindung soll an nachfolgenden Beispielen näher erläutert werden.

### Beispiel 1

In einen Rührkesselreaktor mit einem Volumen von 7 1 wurden kontinuierlich 345 g/h vollentsalztes Wasser und 350 g/h einer Schmelze eines Destillationsrückstands aus der Herstellung von TDI durch Umsetzung von TDA mit Phosgen, der nach SFC-Analyse (Supercritical Fluid Chromatography) noch ca. 20 Gew.-% freies TDI enthielt, über ein Tauchrohr eingeleitet. Die Reaktion wurde bei 200°C und einem konstanten Druck von 30 bar und einem konstanten Füllgrad von 5 l durchgeführt. Die über eine Gasuhr gemessene Gasentwicklung betrug 68,6 l/h. Der Flüssigkeitsaustrag betrug 568 g/h. Die Analyse des Reaktoraustrags ergab einen TDA-Gehalt von 34,1 Gew.-%. Hieraus ergab sich eine TDA-Ausbeute von 55 g TDA pro 100 g eingesetztem TDI-Destillationsrückstand. Das TDA wurde destillativ aus dem Reaktionsgemisch entfernt.

### Beispiel 2

In einen Rührkesselreaktor mit einem Volumen von 2 1 wurden kontinuierlich 34,6 g/h vollentsalztes Wasser und 70,3 g/h einer 50 gew.-%-igen Lösung des TDI-Destillationsrückstandes gemäß Beispiel 1 in Toluol, über ein Tauchrohr eingeleitet. Die Reaktion wurde bei 200°C und einem konstanten Druck von 25 bar und einem konstanten Füllgrad von 800 ml durchgeführt. Die über eine Gasuhr gemessene Gasentwicklung betrug 7,5 l/h. Der Flüssigkeitsaustrag betrug 93 g/h. Die Analyse des Reaktoraustrags ergab einen TDA-Gehalt von 20,6 Gew.-%. Hieraus ergab sich eine TDA-Ausbeute von 54,5 g TDA pro 100 g eingesetztem TDI-Destillationsrückstand.

### Beispiel 3

In einen Rührkesselreaktor wurden kontinuierlich 33,9 g/h einer 5 gew.-%igen wässerigen Natronlauge und 74,4 g/h einer 50 gew.-%igen toluolischen Lösung des TDI-Destillationsrückstandes gemäß Beispiel 1 über ein Tauchrohr eingeleitet. Die Reaktion wurde bei 195°C und einem konstanten Druck von 25 bar und einem konstanten Füllgrad von 800 ml durchgeführt. Die über eine Gasuhr gemessene Gasentwicklung betrug 8,9 l/h. Der Flüssigkeitsaustrag betrug 89,4 g/h. Die Analyse des Reaktoraustrags ergab einen TDA-Gehalt von 25,6 Gew.-%. Hieraus ergab sich eine TDA-Ausbeute von 61,5 g TDA pro 100 g eingesetztem TDI-Destillationsrückstand.

### Beispiel 4

937,4 g TDI-Destillationsrückstand, der nach SFC-Analyse noch ca. 20 Gew.-% freies TDI enthielt, wurde in einem mit rotierenden Teilen versehenen Reaktionsmischer bei 15 mbar und 240°C bis zur Trockene eingeengt. Auf diese Art und Weise wurden 458,6 g Destillat mit einem TDI-Gehalt von 97,3 Gew.-% und 445,7 g eines rieselfähigen, nicht staubenden Rückstandes erhalten. In dem Rückstand ließen sich weder TDI noch NCO-Gruppen nachweisen. Der Bilanzfehler von 33 g ist durch die bei diesen Bedingungen entstehenden gasförmigen Spaltprodukte zu erklären.

In einem Rührkesselreaktor gemäß Beispiel 2 wurden 70 g/h einer bei Raumtemperatur wäßrigen Suspension, die 50 Gew.-% des in Beispiel 4 beschriebenen TDI- und NCO-freien Rückstandes enthielt, über ein Tauchrohr zudosiert. Der Rührkesselreaktor wurde kontinuierlich bei einer Temperatur von 200°C betrieben. Der Austrag von 57,4 g/h enthielt 38,64 Gew.-% TDA. Hieraus ergab sich eine TDA-Ausbeute von 63,4 g TDA pro 100 g eingesetztem TDI-Destillationsrückstand.

### Beispiel 5 (Vergleich)

In einem Rührkessel mit einem Volumen von 2 l wurde 400 g gemahlener TDI-Destillationsrückstand und 400 g VE-Wasser als Suspension vorgelegt. Während des Hochheitzens auf die Reaktionsendtemperatur von 200°C konnte durch ein Schauglas beobachtet werden, wie sich eine feste breiartige Masse bildet, die nicht gerührt werden konnte und die nur langsam abreagierte.

### Beispiel 6 (Vergleich)

In einem Reaktor gemäß Beispiel 2 wurden 300 g Wasser vorgelegt und 600 g einer 50 gew.-%igen Lösung des TDI-Destillationsrückstandes in Toluol mit einer Dosierrate von 2,5 g/h zudosiert. Der Reaktor wurde bei einer Temperatur von 200°C und einem Druck von 25 bar betrieben. Unmittelbar mit der Zudosierung der toluolischen TDI-Destillationsrückstandslösung setzte die Bildung von Feststoff ein. Die Feststoffbildung war so stark, daß der Reaktor nicht mehr durchmischt wurde. Erst nach ca. 3 Stunden baute sich der gebildete Feststoff langsam ab.

### Beispiel 7 (Vergleich)

In einem Reaktor gemäß Beispiel 2 wurden 600 g einer 50 gew.-%igen Lösung des TDI-Destillationsrückstandes in Toluol vorgelegt und 300 g Wasser mit einer Dosierrate von 1,25 g/h zudosiert. Der Reaktor wurde bei einer Temperatur von 200°C und einem Druck von 25 bar betrieben. Unmittelbar mit der Zudosierung des Wassers setzte die Bildung von Feststoff ein. Die Feststoffbildung war so stark, daß der Reaktor nicht mehr durchmischt wurde. Erst nach 1,5 Stunden hatte sich der gebildetet Feststoff abgebaut.

### Beispiel 8

In einem Reaktor gemäß Beispiel 2 wurden 400 g ausreagierter Hydrolyseaustrag gemäß Beispiel 2 vorgelegt und gleichzeitig eine 50 gew.-%ige Lösung des TDI-Destillationsrückstandes in Toluol mit einer Dosierrate von 2,5 g/h und Wasser mit einer Dosierrate von 1,25 g/h zudosiert. Der Reaktor wurde bei einer Temperatur von 200°C und einem Druck von 25 bar betrieben. Unter diesen Bedingungen kam es zu keinerlei Feststoffbildung.

### Beispiel 9

In einem Reaktor gemäß Beispiel 1 wurde kontinuierlich 840 g/h asser und 389 g/h des in Beispiel 1 beschriebenen Destillationsrückstandes zudosiert. Die Reaktion wurde bei 230°C und einem Druck von 35 bar und einem konstanten Füllgrad von 5 l durchgeführt. Die über eine Gasuhr gemessene Gasentwicklung betrug 95 l/h. Der Flüssigkeitsaustrag betrug 1056 g/h. Die Analyse des Reaktoraustrags ergab eine TDA Gehalt von 23,2 gew.-%. Hieraus ergab sich eine TDA-Ausbeute von 63,1 g pro 100 g eingesetztem TDI-Destillationsrückstand.

### Beispiel 10

In einem Reaktor gemäß Beispiel 1 wurde kontinuierlich 830 g/h einer 3 gew.-%igen wässriger Natronlauge und 974 g/h des in Beispiel 1 beschriebenen Destillationsrückstandes zudosiert. Die Reaktion wurde bei 222°C und einem Druck von 35 bar und einem konstanten Füllgrad von 5 l durchgeführt. Die über ein Gasuhr gemessene Gasentwicklung betrug 241 l/h. Der Flüssigkeitsaustrag betrug 1368 g/h. Die Analyse des Reaktoraustrages ergab einen TDa Gehalt von 47,8 gew.-%. Hieraus ergab sich eine TDA-Ausbeute von 67,1 g pro 100 g eingesetztem TDI-Destillationsrückstand.

## Patentansprüche

1. Verfahren zur Aufarbeitung von Destillationsrückständen aus der Synthese von Toluylendiisocyanat (TDI) durch Umsetzung der Destillationsrückstände mit Wasser, **dadurch gekennzeichnet, daß** man die Toluylendiisocyanat-Destillationsrückstände in einem kontinuierlichen oder halbkontinuierlichen Verfahren in einem rückvermischten Reaktor in Gegenwart von Hydrolysat mit Wasser umsetzt, wobei im Reaktionsbehälter Hydrolysat vorgelegt wird, und wobei als Hydrolysat die Umsetzungsprodukte des TDI-Rückständes mit Wasser zu verstehen.

2. Verfahren nach Anspruch 1, **dadurch gekennzeichnet, daß** das halbkontinuierliche Verfahren in einem Rührkessel durchgeführt wird, indem Hydrolysat vorgelegt, zu diesem Hydrolysat Wasser und TDI-Destillationsrückstände zugesetzt werden und nach der Umsetzung das Hydrolysat einer Aufarbeitung zugeführt wird.

3. Verfahren nach Anspruch 1, **dadurch gekennzeichnet, daß** das kontinuierliche Verfahren in einem rückvermischten Reaktor durchgeführt wird.

4. Verfahren nach Anspruch 3, **dadurch gekennzeichnet, daß** als rückvermischter Reaktor ein Rührkessel, eine Rührkesselkaskade, eine Reaktionsmischpumpe, ein Umpumpkreis mit statischem Mischer und/oder Zweistoffmischdüse, ein Strahlschlaufenreaktor oder ein Strahldüsenreaktor eingesetzt wird.

5. Verfahren nach Anspruch 1, **dadurch gekennzeichnet, daß** die Hydrolyse bei einer Temperatur im Bereich von 120°C bis 250°C durchgeführt wird.

6. Verfahren nach Anspruch 1, **dadurch gekennzeichnet, daß** die Hydrolyse bei einer Temperatur im Bereich von 170°C bis 230°C durchgeführt wird.

7. Verfahren nach Anspruch 1, **dadurch gekennzeichnet, daß** die Hydrolyse bei einem Druck im Bereich von 1 bis 50 bar durchgeführt wird und der Druck höher ist als der Siededruck des Austrags aus dem Reaktor bei der Reaktionstemperatur.

8. Verfahren nach Anspruch 1, **dadurch gekennzeichnet, daß** die TDI-Destillationsrückstände in die Flüssigphase des Reaktors zugegeben werden.

9. Verfahren nach Anspruch 1, **dadurch gekennzeichnet, daß** die Hydrolyse in Anwesenheit von Basen oder Säuren durchgeführt wird.

10. Verfahren nach Anspruch 1, **dadurch gekennzeichnet, daß** die Hydrolyse in Anwesenheit von Salzen von Eisen, Zink, Zinn und Zirkon durchgeführt wird.

11. Verfahren nach Anspruch 1, **dadurch gekennzeichnet, daß** das Massenverhältnis von zugeführten TDI-Destillationsrückständen zu zugeführtem Wasser bei der Hydrolyse im Bereich zwischen 4,8:1 und 1:5 liegt.

12. Verfahren nach Anspruch 1, **dadurch gekennzeichnet, daß** das Massenverhältnis von zugeführtem TDI-Destillationsrückstand zu zugeführtem Wasser im Bereich zwischen 1:1,0 und 1:3 liegt.

13. Verfahren nach Anspruch 1, **dadurch gekennzeichnet, daß** die TDI-Destillationsrückstände in einem organischen Lösemittel, das mit den TDI-Rückständen nicht reagiert, gelöst werden.

14. Verfahren nach Anspruch 1, **dadurch gekennzeichnet, daß** das Hydrolysat ohne weitere Aufarbeitung nach bekannten Verfahren durch Umsetzung mit Alkylenoxiden zu Polyetheralkoholen umgesetzt wird.

15. Verfahren zur Herstellung von TDI durch Umsetzung von Toluylendiamin (TDA) mit Phosgen und anschließende destillative Abtrennung des gebildeten TDI, **dadurch gekennzeichnet, daß** die verbleibenden Destillationsrückstände einer Hydrolyse gemäß Anspruch 1 unterzogen werden, aus dem Hydrolysat das TDA abtrennt und dieses wiederum der Phosgenierung zuführt.

## Claims

1. A process for working up distillation residues from the synthesis of tolylene diisocyanate (TDI) by reaction of the distillation residues with water, which comprises reacting the tolylene diisocyanate distillation residues with water in a continuous or semicontinuous process in a backmixed reactor in the presence of hydrolysate, the reaction vessel being initially charged with hydrolysate and hydrolysate referring to the reaction products of the TDI residue with water.

2. A process as claimed in claim 1, wherein the semicontinuous process is carried out in a stirred tank by initially charging the tank with hydrolysate, adding water and TDI distillation residues to this hydrolysate and working up the hydrolysate after the raction.

3. A process as claimed in claim 1, wherein the continuous process is carried out in a backmixed reactor.

4. A process as claimed in claim 3, wherein the backmixed reactor is a stirred tank, a stirred tank cascade, a reaction mixing pump, a pumped circuit provided with a static mixer and/or a two-fluid mixing nozzle, a jet loop reactor or a jet nozzle reactor.

5. A process as claimed in claim 1, wherein the hydrolysis is carried out at from 120°C to 250°C.

6. A process as claimed in claim 1, wherein the hydrolysis is carried out at from 170°C to 230°C.

7. A process as claimed in claim 1, wherein the hydrolysis is carried out at a pressure in the range from I to 50 bar and the pressure is higher than the boiling pressure of the product discharged from the reactor at the reaction temperature.

8. A process as claimed in claim 1, wherein the TDI distillation residues are introduced into the liquid phase of the reactor.

9. A process as claimed in claim 1, wherein the hydrolysis is carried out in the presence of bases or acids.

10. A process as claimed in claim 1, wherein the hydrolysis is carried out in the presence of salts of ron@, zinc, tin or zirconium.

11. A process as claimed in claim 1, wherein the mass ratio of TDI distillation residues introduced to water introduced in the hydrolysis is from 4.8:1 to 1:5.

12. A process as claimed in claim 1, wherein the mass ratio of TDI distillation residue introduced to water introduced is from 1:1.0 to 1:3.

13. A process as claimed in claim 1, wherein the TDI distillation residues are dissolved in an organic solvent which does not react with the TDI residues.

14. A process as claimed in claim 1, wherein the hydrolysate is, without further work-up, reacted by known methods with alkylene oxides to form polyether alcohols.

15. A process for preparing TDI by reacting tolylenediamine (TDA) with phosgene and subsequently separating off the resulting TDI by distillation, which comprises subjecting the remaining distillation residues to a hydrolysis as claimed in claim 1, separating the TDA from the hydrolysate and returning this to the phosgenation.

## Revendications

1. Procédé pour le traitement de résidus de distillation provenant de la synthèse du toluylènediisocyanate (TDI) par mise en réaction des résidus de distillation avec de l'eau, **caractérisé en ce qu'**on fait réagir les résidus de distillation de toluylènediisocyanate, dans un procédé en continu ou en semi-continu dans un réacteur soumis à un remélangeage en présence d'un hydrolysat avec de l'eau, dans lequel on dépose l'hydrolysat au préalable dans le récipient de réaction et dans lequel on entend par hydrolysat les produits réactionnels du résidu de TDI avec de l'eau.

2. Procédé selon la revendication 1, **caractérisé en ce qu'**on effectue le procédé en semi-continu dans une cuve d'agitation dans laquelle on dépose l'hydrolysat au préalable, on ajoute de l'eau et des résidus de distillation de TDI à cet hydrolysat et après la mise en réaction, on achemine l'hydrolysat à un traitement.

3. Procédé selon la revendication 1, **caractérisé en ce qu'**on effectue le procédé en continu dans un réacteur soumis à un remélangeage.

4. Procédé selon la revendication 3, **caractérisé en ce qu'**on met en oeuvre, à titre de réacteur soumis à un remélangeage, une cuve d'agitation, une cascade de cuves d'agitation, une pompe du mélange réactionnel, un circuit de transvasement par pompe comprenant un mélangeur statique et/ou une buse de mélange de deux substances, un réacteur à jet à écoulement en boucles ou encore un réacteur à jet.

5. Procédé selon la revendication 1, **caractérisé en ce qu'**on effectue l'hydrolyse à une température dans la plage de 120 °C à 250 °C.

6. Procédé selon la revendication 1, **caractérisé en ce qu'**on effectue l'hydrolyse à une température dans la plage de 170 °C à 230 °C.

7. Procédé selon la revendication 1, **caractérisé en ce qu'**on effectue l'hydrolyse sous une pression dans la plage de 1 à 50 bars, la pression étant supérieure à la pression d'ébullition du produit d'évacuation du réacteur à la température de réaction.

8. Procédé selon la revendication 1, **caractérisé en ce qu'**on ajoute les résidus de distillation de TDI à la phase liquide du réacteur.

9. Procédé selon la revendication 1, **caractérisé en ce qu'**on effectue l'hydrolyse en présence de bases ou d'acides.

10. Procédé selon la revendication 1, **caractérisé en ce qu'**on effectue l'hydrolyse en présence de sels de fer, de zinc, d'étain et de zirconium.

11. Procédé selon la revendication 1, **caractérisé en ce que** le rapport de masse des résidus de distillation de TDI acheminés à l'eau acheminée lors de l'hydrolyse se situe dans la plage entre 4,8 : 1 et 1 : 5.

12. Procédé selon la revendication 1, **caractérisé en ce que** le rapport de masse du résidu de distillation de TDI acheminé à l'eau acheminée se situe dans la plage entre 1 : 1,0 et 1 : 3.

13. Procédé selon la revendication 1, **caractérisé en ce que** les résidus de distillation de TDI sont dissous dans un solvant organique qui ne réagit pas avec les résidus de TDI.

14. Procédé selon la revendication 1, **caractérisé en ce qu'**on fait réagir l'hydrolysat sans traitement ultérieur conformément à des procédés connus par mise en réaction avec des oxydes d'alkylènes pour obtenir des polyéther-alcools.

15. Procédé pour la préparation de TDI par mise en réaction de toluylènediamine (TDA) avec du phosgène et par séparation ultérieure par distillation du TDI obtenu, **caractérisé en ce qu'**on soumet à une hydrolyse selon la revendication 1 les résidus de distillation qui subsistent, on sépare la TDA de l'hydrolysat et on le renvoie à la phosgénation.
